# EUROPÄISCHE PATENTANMELDUNG

(11) **EP 1 273 888 A2**
(43) Veröffentlichungstag der Anmeldung: **08.01.2003**
(21) Anmeldenummer: 02013939.0
(22) Anmeldetag: 24.06.2002
(51) Int. Cl.: G01D 4/00

(54) **Erfassungseinrichtung für ein Fluid und Verfahren zur Ermittlung des Wechsels einer Fluidart**

(30) Priorität: 03.07.2001 DE 10132477
(71) Anmelder: SIEMENS AKTIENGESELLSCHAFT, 80333 München (DE)
(72) Erfinder: Gärtner, Franz-Georg, Dr., 90461 Nürnberg (DE)

(57) **Zusammenfassung**

Um auf besonders einfache Weise eine Ermittlung des Wechsels einer Fluidart zu erzielen, ist vorgesehen, dass eine Erfassungseinrichtung eine physikalische Eigenschaft des Fluids ermittelt. Bei einer Änderung der physikalischen Eigenschaft wird von einer Änderung der Fluidart ausgegangen. Es wird dann eine Zeitinformation abgespeichert. Optional kann das zugehörige Fluidvolumen ermittelt werden. Beide Informationen können dann für eine Verrechnung und Berechnung des Heizwertes an eine Zentrale übermittelt werden.

## Beschreibung

Bei heutigen Gaszählern für Haushalte wird in der Regel am Verbrauchsort ein Gasvolumen, z.B. in m³, erfasst. Für die Erfassung des Gasvolumens kommt gemäß moderner Technik z.B. ein Ultraschalldurchflusszähler zum Einsatz. Das Gasversorgungsunternehmen (GVU) muss jedoch gegenüber dem Kunden die verbrauchte Energie oder den Brennwert, z.B. in Kwh, abrechnen. Dazu wird das vor Ort gemessene Gasvolumen unter zur Hilfenahme von ermittelten oder angenäherten Größen (Druck, Temperatur, Gasart) zentral beim GVU und zeitabhängig auf den Energieinhalt umgerechnet.

Wenn in einem Energieversorgungsnetz die Gasart (in der Regel ein Gemisch) z.B. durch einen Lieferantenwechsel gewechselt wird, (was in der Praxis ca. 4 mal pro Jahr erfolgen kann) so muss dies bei der Berechnung der verbrauchten Gasenergie durch den Brennwert pro Mol rechnerisch berücksichtigt werden. Dazu wird aus dem Zeitpunkt der zentralen Einspeisung, über Erfahrungswerte und mathematische Modelle der Gasgemischausbreitung der Eintreffzeitpunkt der neuen Gasgruppe an der Verbrauchstelle beim Verbraucher errechnet.

Dieses Verfahren umfasst zur Ermittlung der verbrauchten Gasenergie nur eine einzige Vorort-Messgröße. Alle anderen physikalischen Größen und die zeitlichen Veränderungen werden durch Mittelwerte und mathematischen Methoden ersetzt. Dies führt dazu, dass entsprechend dem derzeit "wirtschaftlich machbaren" allenfalls eine im Jahresmittel "gerechte" Abrechnung entsteht.

Mit der Liberalisierung des Energiemarktes, die auch zukünftig die Gasversorgung betrifft, ist die oben beschriebene Verfahrensweise nicht genau genug. Es wird erwartet, dass zukünftig etwa 20 % der Gasverbraucher ihr GVU mehrmals jährlich wechseln werden, um einen günstigen Gastarif zu nutzen. Die Vertragslaufzeiten werden dann nicht mehr Jahre, sondern nur noch Quartale oder Monate umfassen.

Das Verfahren zur Abrechnung der verbrauchten Gasenergie muss daher zumindest "monatsgenau" sein.

Aus der WO 99/49299 ist eine Einrichtung zur Ermittlung des Energiegehalts eines Fluids, insbesondere Gas bekannt, bei der die Durchflussmenge durch eine Erfassungseinrichtung mittels Ultraschall festgestellt wird. Zusätzlich ist eine Einrichtung zur Detektion der im Gas enthaltenen Gasarten und ihrer Volumina vorgesehen. Auf Basis dieser Werte und der zugehörigen Energiewerte sowie der entsprechenden Durchflussmenge wird dann der Energiegehalt des die Messeinrichtung durchfließenden Fluids für einen vorgegebenen Zeitraum berechnet. Diese Einrichtung ist relativ aufwendig, da die Gasarten im einzelnen ermittelt werden.

Der Erfindung liegt die Aufgabe zugrunde, eine Einrichtung und ein zugehöriges Verfahren zur Ermittlung des Wechsels einer Fluidart anzugeben, die bzw. das besonders einfach ist und aufbauend auf herkömmlichen Geräten auf einfache Weise hergestellt werden kann.

Die Lösung der Aufgabe gelingt erfindungsgemäß mit den Merkmalen des Anspruchs 1. Ausgangsgedanke der Erfindung ist es, dass eine genaue Abrechnung auf Basis der entsprechenden Gasart, Gasgemisches oder Fluidsorte erfolgen soll. Dazu wird vor Ort ein Zeitpunkt für die Änderung der Fluidsorte ermittelt. Die Erfindung umfasst demnach folgende Merkmale:

Erfassungseinrichtung für ein Fluid, insbesondere für Gas, mit einer Einrichtung zur Erfassung zumindest einer physikalischen Eigenschaft des Fluids und einer Recheneinrichtung mit Speicher, wobei
- auf Basis der von der Einrichtung gelieferten Messwerte zumindest eine physikalische Eigenschaft oder eine Kombination physikalischer Eigenschaften des Fluids zu einem ersten und einem zweiten Zeitpunkt ermittelt werden,
- diese Messwerte miteinander verglichen werden, und
- bei einer eine vorgegebene Grenze unter- oder überschreitenden Differenz zwischen den Messwerten eine Zeitinformation im Speicher gespeichert ist.

Diese Einrichtung lässt sich einerseits als separates Gerät, z.B. für die Nachrüstung, mit bereits vorhandenen Durchflussmessern kombinieren. Dies können beispielsweise herkömmliche mechanische oder auf dem Ultraschallmessprinzip beruhende sein. Andererseits ist auch eine Integration in einen Durchflussmesser möglich.

Bevorzugt umfasst die Erfassungseinrichtung eine IR-Absorbtionsmesseinrichtung (IR = Infrarot) wobei als zu vergleichende Messwerte Werte der Infrarotabsorbtionsbanden des Fluids dienen. Diese lässt sich mit relativ wenig Aufwand, zum Beispiel mittels einer Infrarotlichtschranke, realisieren.

Die Erfassungseinrichtung kann auch eine Ultraschall-Messeinrichtung umfassen, wobei als zu vergleichende Messwerte Werte der Ultraschallausbreitungsgeschwindigkeit im Fluid dienen. Diese Technik lässt sich einfach installieren und ist in der Gas-, Wärme- und Wasserzähltechnik seit langem erprobt.

Alternativ oder zusätzlich kann die Erfassungseinrichtung eine Wärmeleitfähigkeits-Messeinrichtung umfassen. Dabei dienen als zu vergleichende Messwerte Werte der Wärmeleitfähigkeit des Fluids. Diese Ausführung lässt sich kostengünstig mit wenig Aufwand herstellen.

Es ist günstig, wenn die Erfassungseinrichtung eine Dielektrizitätskonstanten-Messeinrichtung umfasst und als zu vergleichende Messwerte Werte der Dielektrizitätskonstanten des Fluids dienen. Es kann weiterhin eine Einrichtung zur Erfassung der elektrischen Leitfähigkeit vorgesehen sein , wobei als zu vergleichende Messwerte Werte der elektrischen Leitfähigkeit des Fluids dienen. Für diese Ausführungen können bekannte oder auf dem Markt vorhandene Techniken verwendet werden.

Mit Vorteil kann optional eine Volumenmesseinrichtung vorgesehen sein, wobei dann zusätzlich zur Zeitinformation ein Wert für ein kumuliertes Fluidvolumen gespeichert ist. Damit ist ein Wertpaar gegeben, das gemeinsam für die Abrechnung des Energie- oder Brennwertverbrauchs genutzt werden kann. Somit ist auch quasi auf Basis eines einfachen Durchflussmessers oder Wärmezählers als Volumenmesseinrichtung eine genaue Abrechnung eines Energieverbrauchs möglich. Bevorzugt kommt dabei ein Ultraschalldurchflussmesser zur Anwendung. Zusätzliche Funktionalitäten können ggf. mit geringem Aufwand in einen solchen herkömmlichen Ultraschalldurchflussmesser integriert werden. Dies betrifft insbesondere die Ermittlung und Speicherung der Messwerte, die als Programm im Speicher der Recheneinrichtung zugeordnet sein können. Eine Verarbeitung der so ermittelten Werte kann dann zentral beim EVO oder Gasversorgungsunternehmen erfolgen.

Bevorzugt ist/sind ein Display und/oder eine Schnittstelle vorgesehen, wobei zumindest die gegebenenfalls gespeicherten Werte für die Zeitinformation und das Fluidvolumen über das Display und/oder die Schnittstelle auslesbar und gegebenenfalls an eine Zentrale übertragbar. Somit ist eine Ablesbarkeit vor Ort oder auch eine Übertragung an ein Auslesemedium, z.B. ein PC, oder auch eine Fernübertragung an eine zentrale Recheneinrichtung möglich.

Erfindungsgemäß ist zur Lösung der Aufgabe weiterhin ein Verfahren zur Ermittlung des Wechsels einer Fluidart, insbesondere von Gas, mit folgenden Schritten vorgesehen:
- an einem Messort wird zu einem ersten Zeitpunkt ein erster Messwert für zumindest eine physikalische Eigenschaft des Fluids ermittelt und gespeichert,
- zu einem zweiten Zeitpunkt wird für die physikalische Eigenschaft ein zweiter Wert ermittelt und gespeichert,
- die beiden Messwerte für die physikalische Eigenschaft werden miteinander verglichen,
- bei einer eine vorgegebene Grenze unter- oder überschreitenden Differenz zwischen den Messwerten wird eine Zeitinformation als Information für den Wechsel der Gasart gespeichert.

Die oben genannten Vorteile der Erfassungseinrichtung gelten hier sinngemäß. Mit Vorteil werden die Schritte zu weiteren Zeitpunkten fortlaufend wiederholt. Damit ist ein kontinuierlicher Prozess gegeben, der eine dauerhafte Überwachung erlaubt.

Dabei kann ein Wert für das den Messort zwischen den beiden Zeitpunkten passierende Fluidvolumen ermittelt und gespeichert werden. Somit ist mit der Zeitinformation ein Wertepaar gegeben, auf dessen Basis eine genaue Energieabrechnung für einen Kunden erstellt werden kann.

Die Speicherung von Zeitinformation und Fluidvolumen kann mit Vorteil in einem Gerät am ersten Messort erfolgen, wobei diese Werte an einen zweiten Ort übertragen und dort der Brennwert für das jeweilige Fluidvolumen ermittelt wird. Somit ist eine räumliche Trennung gegeben, die eine dezentrale Informationserfassung mit zentraler Berechnung ermöglicht.

Weitere vorteilhafte Ausgestaltungen sind in den übrigen Ansprüchen angegeben.

Weitere Vorteile und Details der Erfindung werden nachfolgend anhand eines Ausführungsbeispiels näher erläutert.

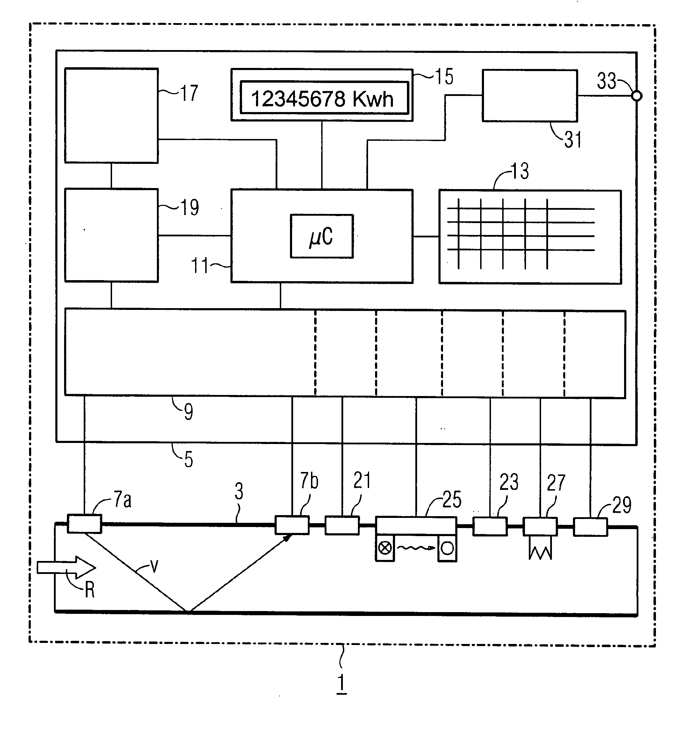

Die einzige Figur zeigt eine Ausführungsform einer Erfassungseinrichtung, die als Durchflusszähler 1 für ein Fluid ausgebildet ist. Unter Fluid wird vorliegend insbesondere verstanden: Ein Gas, ein Gas- oder ein flüssiges Brennstoffgemisch oder ein flüssiger Brennstoff. Gasgemische werden üblicherweise auch als Gasgruppen bezeichnet, hier jedoch allgemein als Gas bezeichnet.

Der Durchflusszähler 1 umfasst prinzipiell eine Volumenmesseinrichtung ein Ultraschallmessrohr - nachfolgend als Messrohr 3 bezeichnet - und eine zugehörige Elektronik 5. Das Messrohr 3 arbeitet beispielsweise nach dem allgemein bekannten Ultraschalllaufzeitprinzip, wobei ausgehend von einem mittels einem ersten Schallkopf 7a ausgesendeten Ultraschallsignal auf einem Weg V durch das im Messrohr 3 fließende Medium oder Fluid geführt und von einem zweiten Schallkopf 7b empfangen wird. Dieser Vorgang wird in entgegengesetzter Richtung wiederholt. Auf Basis der Laufzeitunterschiede wird dann die Fließgeschwindigkeit des Fluids in Richtung R ermittelt. Der Durchflusszähler 1 kann selbstverständlich auch mit einer mechanischen Zähleinrichtung, z.B. mit Flügelrad, versehen sein.

Zu den verschiedensten Ausführungen bezüglich Schallkopfanordnung, Messrohrstrecke und Schallweg wird auf den bekannten Stand der Technik verwiesen. Ein als Messeinrichtung dienendes Eingangsmodul 9, das nicht näher gezeigte Sende- und Empfangsmittel umfasst, ist über nicht näher bezeichnete Leitungen elektrisch mit den Schallköpfen 7a und 7b verbunden.

Das Eingangsmodul 9 ist ausgangsseitig mit einer zentralen Recheneinrichtung 11 verbunden, die insbesondere einen Mikroprozessor umfasst. Diesem ist ergänzend ein Speicher 13, eine Anzeigevorrichtung, insbesondere ein Display 15, ein Bedienmodul, insbesondere eine Tastatur 17, und ein Energieversorgungsmodul, insbesondere ein Netzteil oder eine Batterie 19, zugeordnet. Tastatur 17 und Display 15 dienen zum Bedienen und zum Informationsaustausch. Die Batterie 19 dient zur Energieversorgung der gesamten Erfassungseinrichtung.

In dem Speicher 13 sind unter anderem übliche Betriebsprogramme für den Betrieb des Durchflusszählers 1 abgelegt. In der Recheneinrichtung 11 werden die von dem Eingangsmodul 9 gelieferten Signale oder Werte verarbeitet.

Zusätzlich kann die gesamte Anordnung als Einrichtung zur Erfassung zumindest einer physikalischen Eigenschaft noch folgende Sensoren, die entweder direkt am Messrohr 3 oder zumindest in Nähe des Messrohres 3 am Fluidstrom angeordnet sein müssen, alternativ oder in beliebiger Kombination optional umfassen:

Einen Temperatursensor 21, einen Drucksensor 23, eine Infrarot(IR)-Absorbtionsmesseinrichtung 25, die beispielsweise eine Infrarotlichtschranke umfasst, gebildet sein kann, einen heizbaren Widerstand 27 oder einen Sensor 29 zur Erfassung der Leitfähigkeit oder einer Dielektrizitätskonstanten des Fluids.

Diese Sensoren können entweder ebenfalls mit dem Eingangsmodul 9 oder mit gegebenenfalls separaten Eingangsmodulen oder Anpassbaugruppen an die Rechnereinrichtung 11 angeschlossen sein. Vorliegend sind alle Sensoren über ein gemeinsames Eingangsmodul 9 mit der Rechnereinrichtung 11 verbunden. Die Modularität oder getrennte Verarbeitung der Messwerte oder Signale ist durch die strichlierte Unterteilung des Eingangsmoduls 9 angedeutet. Der Temperatursensor 21 und der Drucksensor 23 werden gegebenenfalls benötigt, falls eine Wärmezählung in der gesamten Anordnung durchgeführt wird.

Zur weiteren Kommunikation oder zur Ablesung kann die Rechnereinrichtung 11 über ein Schnittstellenmodul 31 mit einem Ausgang 33 verbunden sein. Der Ausgang 33 kann als elektrische Verbindung oder auch als optische Schnittstelle ausgebildet sein, so dass die von der Rechnereinrichtung 11 erzeugten oder gelieferten Werte ausgelesen werden können. Gegebenenfalls kann das Schnittstellenmodul 31 auch zur Eingabe von Informationen in die Rechnereinrichtung 11 verwendbar sein.

Somit sind zunächst alle wesentlichen Hardwarekomponenten des Durchflusszählers 1 aufgezeigt. Nachfolgend wird auf die Funktion im Detail eingegangen.

Bei der neuen Erfassungseinrichtung und dem zugehörigen Verfahren wird von dem Grundgedanken ausgegangen, dass eine Änderung der Fluidart oder im speziellen Fall der Gasart ermittelt wird. Dazu wird zumindest eine charakteristische physikalische Eigenschaft des Fluids überwacht.

Dies kann durch eine optische Methode, beispielsweise mittels der IR-Absorbtionsmesseinrichtung 25, erfolgen, bei der die Infrarotlichtabsorption oder die Infrarotlichtabsorptionsbanden des Gases als physikalische Eigenschaft ermittelt wird/werden. Dabei wird zu einem ersten und einem zweiten Zeitpunkt jeweils eine Messung vorgenommen. Diese Messung kann auch als zu in vorgegebenen Zeitpunkten sich wiederholende, fortlaufende Messwerterfassung vorgesehen sein. Die jeweils aufeinanderfolgenden Werte werden dann miteinander verglichen. Geht eine Änderung unter oder über einen vorgegeben Grenzwert oder außerhalb eines Grenzwertbandes hinaus, so wird auf eine Änderung der Fluidart geschlossen. Die Grenzwertverletzung wird also als Indikator für den Fluidwechsel verwendet.

Der Zeitpunkt der Änderung (Relativ- oder Realzeit) wird dann ggf. gemeinsam mit einem kumuliertem Wert des Fluidvolumens als Wertpaar im Speicher 13 hinterlegt. Die Grenze oder die Grenzbandbedingungen sind ggf. änderbar im Speicher 13 hinterlegt und können ggf. über die Tastatur 17 oder über das Schnittstellenmodul 31 von extern geändert oder vorgegeben werden.

Dieser vergleichende Prozess kann auch eine Verfahrensweise umfassen, bei der beispielsweise eine Unstetigkeit in dem Messsignal oder die zeitliche Änderung des Messsignals der IR-Absorbtionsmesseinrichtung 25 ermittelt wird. Tritt eine Unstetigkeit oder überproportionale Änderung im Messsignal oder -wert auf, so wird die Zeitinformation oder eine Zeitmarke im Speicher 13 hinterlegt.

Selbstverständlich kann die Erfassung der Fluidartänderung auf verschiedene physikalische Eigenschaften beruhen. Hierzu können alle oben beschriebenen und geeigneten Sensoren, die am Messrohr 3 angeordnet sind, beliebig verwendet werden.

Eine weitere oder alternative Ausführungsart sieht demnach vor, dass mit einer Ultraschallmesseinrichtung die Ultraschall-Ausbreitungsgeschwindigkeit im Fluid ermittelt wird. Somit würde als Indikator für eine Fluidartänderung die Schallausbreitungsgeschwindigkeit im Fluid zur Verfügung stehen. Bei Vorhandensein eines Durchflussmessers auf Basis von Ultraschall kann dieser quasi als Messwertgeber dienen.

Folgende Messeinrichtungen eignen beispielsweise ebenfalls zur Erfassung einer physikalischen Eigenschaft:
- Eine Wärmeleitfähigkeits-Messeinrichtung zur Erfassung der Wärmeleitfähigkeit des Fluids. Hierzu kann der heizbare Widerstand 27 dienen.
- Eine Messeinrichtung zur Erfassung der elektrischen Leitfähigkeit des Fluids. Hierzu kann der Sensor 29 dienen.
- Eine Dielektrizitätskonstanten-Messeinrichtung zur Erfassung der Dielektrizitätskonstanten des Fluids. Hierzu kann ebenfalls der Sensor 29 dienen

Zusätzlich kann - wie bereits oben angesprochen - die Zeitinformation mit einer Information über das Fluidvolumen, das bis zu dem Zeitpunkt das Messrohr 3 durchflossen hat versehen werden. Damit steht ein Wertepaar für eine genaue Berechnung zur Verfügung.

Die Bearbeitung der Messwerte und Erfassung der Änderungen erfolgt mittels eines geeigneten Programms, welches im Speicher 13 hinterlegt ist, in Verbindung -mit der Recheneinrichtung 11, die als Mikroprozessor, gegebenenfalls auch innerhalb eines Asics, ausgeführt sein kann.

Die Verarbeitung der so erzeugten Informationen und Messwerte kann vor Ort durch eine manuelle Ablesung oder durch Datenübertragung erfolgen. Dabei kann beispielsweise Bedienpersonal mittels eines Ablesegerätes am Ausgang 33 die so erzeugten Daten, insbesondere die Wertpaare für einen vorgegebenen Zeitraum, übernehmen und diese zur Einspielung in einen Zentralrechner übergeben. Selbstverständlich ist auch eine direkte Übertragung vom Ausgang 33 an eine zentrale Abrechnungsstelle denkbar. Für das gesamte Verfahren kann demnach eine örtliche Aufteilung der Verfahrensschritte in Frage kommen.

Das bevorzugte Anwendungsgebiet für die neue Erfassungseinrichtung und das Verfahren ist die Abrechnung eines Gasverbrauchs für Endkunden, insbesondere für Haushalte. Günstig ist dabei, dass dezentral keinerlei Vergleichswerte oder Informationen für die Gase, Gasgemische oder absolute relative Energiewerte abgespeichert sein müssen. Im lokal angeordneten Zähler beim Kunden wird bevorzugt lediglich ein Volumenverbrauch und eine Änderung des Fluids ermittelt. Die absolute Berechnung erfolgt dann mit zentral gespeicherten Werten bei der Abrechnungsstelle.

Dies ist insbesondere dann günstig, wenn gegebenenfalls völlig neue Gasgemische von neuen Zulieferern in ein Netz eingespeist werden. In einem solchen Fall müssten nämlich alle Geräte vor Ort mit neuen Informationen über die Gasart versehen werden. Bei der neuen Idee muss lediglich die Information des Eintreffens der neuen Gassorte beim Kunden mit dem zugehörigen Verbrauchsvolumen zentral übermittelt werden.

Selbstverständlich ist es möglich, dass für einen größeren Abrechnungszeitraum, z. B. 1 Jahr, im Speicher 13 mehrere Wertepaare für verschiedene Gaswechsel abgelegt werden.

Prinzipiell ist dieses Verfahren auf alle Fluide anwendbar, bei denen insbesondere ein Fluidwechsel in Verbindung mit einer Volumenmessung erfasst werden soll. Dies geht also über die eigentliche Energiezählung hinaus. Speziell ist jedoch auch eine Anwendung bei flüssigen Fluiden, z. B. flüssige Brennstoffe, denkbar, wobei dann jedoch gegebenenfalls bei der Erfassung der physikalischen Eigenschaft hinsichtlich der Brennbarkeit oder Explosionssicherheit andere Randbedingungen eingehalten werden müssen. Bevorzugte Anwendung ist die Verbrauchsmessung von Gasgemischen.

## Patentansprüche

1. Erfassungseinrichtung für ein Fluid, insbesondere für Gas, mit einer Einrichtung zur Erfassung zumindest einer physikalischen Eigenschaft des Fluids und einer Recheneinrichtung mit Speicher, wobei
- auf Basis der von der Einrichtung gelieferten Messwerte zumindest eine physikalische Eigenschaft oder eine Kombination physikalischer Eigenschaften des Fluids zu einem ersten und einem zweiten Zeitpunkt ermittelt werden,
- diese Messwerte miteinander verglichen werden, und
- bei einer eine vorgegebene Grenze unter- oder überschreitenden Differenz zwischen den Messwerten eine Zeitinformation im Speicher gespeichert ist.

2. Erfassungseinrichtung nach Anspruch 1, wobei die Einrichtung eine IR-Absorbtionsmesseinrichtung umfasst und als zu vergleichende Messwerte Werte der Infrarotabsorbtionsbanden des Fluids dienen.

3. Erfassungseinrichtung nach einem der Ansprüche 1 oder 2, wobei die Erfassungseinrichtung eine Ultraschallmesseinrichtung umfasst und als zu vergleichende Messwerte Werte der Ultraschallausbreitungsgeschwindigkeit im Fluid dienen.

4. Erfassungseinrichtung nach einem der Ansprüche 1 bis 3, wobei die Erfassungseinrichtung eine Wärmeleitfähigkeits-Messeinrichtung umfasst und als zu vergleichende Messwerte Werte der Wärmeleitfähigkeit des Fluids dienen.

5. Erfassungseinrichtung nach einem der Ansprüche 1 bis 4, wobei die Erfassungseinrichtung eine Dielektrizitätskonstanten-Messeinrichtung umfasst und als zu vergleichende Messwerte Werte der Dielektrizitätskonstanten des Fluids dienen.

6. Erfassungseinrichtung nach einem der Ansprüche 1 bis 5, wobei die Erfassungseinrichtung eine Einrichtung zur Erfassung der elektrischen Leitfähigkeit umfasst und als zu vergleichende Messwerte Werte der elektrischen Leitfähigkeit des Fluids dienen.

7. Erfassungseinrichtung nach einem der Ansprüche 1 bis 6, wobei eine Volumenmesseinrichtung vorgesehen ist und zusätzlich zur Zeitinformation ein Wert für ein kumuliertes Fluidvolumen gespeichert ist.

8. Erfassungseinrichtung nach einem der Ansprüche 1 bis 7, wobei ein Display und/oder eine Schnittstelle vorgesehen ist/sind und zumindest die gespeicherten Werte für die Zeitinformation und das Fluidvolumen über das Display und/oder die Schnittstelle auslesbar und gegebenenfalls an eine Zentrale übertragbar sind.

9. Verfahren zur Ermittlung des Wechsels einer Fluidart, insbesondere von Gas, mit folgenden Schritten:
- an einem Messort wird zu einem ersten Zeitpunkt ein erster Messwert für zumindest eine physikalische Eigenschaft des Fluids ermittelt und gespeichert,
- zu einem zweiten Zeitpunkt wird für die physikalische Eigenschaft ein zweiter Wert ermittelt und gespeichert,
- die beiden Messwerte für die physikalische Eigenschaft werden miteinander verglichen,
- bei einer eine vorgegebene Grenze unter- oder überschreitenden Differenz zwischen den Messwerten wird eine Zeitinformation als Information für den Wechsel der Gasart gespeichert.

10. Verfahren nach Anspruch 9, wobei die Schritte zu weiteren Zeitpunkten fortlaufend wiederholt werden.

11. Verfahren nach Anspruch 9 oder 10, wobei ein Wert für das den Messort zwischen den beiden Zeitpunkten passierende Fluidvolumen ermittelt und gespeichert wird.

12. Verfahren nach Anspruch 11, wobei die Speicherung von Zeitinformation und Fluidvolumen in einem Gerät am ersten Messort erfolgt, diese Werte an einen zweiten Ort übertragen und dort der Brennwert für das Fluidvolumen ermittelt wird.
